# FASCICULE DE BREVET EUROPEEN

(11) **EP 3 697 483 B1**
(45) Date de publication et mention de la délivrance du brevet: **08.12.2021**
(21) Numéro de dépôt: 18815269.8
(22) Date de dépôt: 18.10.2018
(51) Int. Cl.: A61M 15/00

(54) **DISPOSITIF DE DISTRIBUTION DE PRODUIT FLUIDE SYNCHRONISE AVEC L'INHALATION**
VORRICHTUNG ZUR INHALATIONSSYNCHRONISIERTEN ABGABE EINES FLÜSSIGEN PRODUKTS
DEVICE FOR INHALATION-SYNCHRONIZED DISPENSING OF A FLUID PRODUCT

(30) Priorité: 18.10.2017 FR 1759800
(43) Date de publication de la demande: 26.08.2020
(73) Titulaire: APTAR France SAS, 27110 Le Neubourg (FR)
(72) Inventeur: FABIEN, David, 22130 Corseul (FR)
(74) Mandataire: CAPRI
(86) Numéro de dépôt international: PCT/FR2018/052594
(87) Numéro de publication internationale: WO 2019/077276

(56) Documents cités:
- WO-A1-2013/038169
- WO-A1-2017/176704
- WO-A2-2006/115732
- US-A- 3 456 646
- US-A- 5 119 806

## Description

La présente invention concerne un dispositif de distribution de produit fluide synchronisé avec l'inhalation, et plus particulièrement un dispositif d'inhalation du type aérosol synchronisé avec l'inhalation.

Les dispositifs actionnés par l'inhalation, désignés généralement par le terme B.A.I. (signifiant "Breath Actuated Inhaler"), sont bien connus dans l'état de la technique. L'avantage principal de ce type de dispositif est que la distribution du produit est synchronisée avec l'inhalation du patient, pour garantir une bonne distribution du produit dans les voies respiratoires. Ainsi, dans le domaine des dispositifs aérosol, c'est-à-dire ceux dans lesquels le produit est distribué à l'aide d'un gaz propulseur, de nombreux types de dispositifs de déclenchement par l'inhalation ont été proposés. Ces dispositifs présentent toutefois l'inconvénient de comporter un grand nombre de pièces, c'est-à-dire qu'ils sont compliqués et coûteux à fabriquer et à assembler, ce qui est évidemment désavantageux. Il est aussi difficile de trouver le bon équilibre entre un déclenchement fiable à chaque inhalation, sans que le seuil de déclenchement ne soit trop élevé, et une serrure suffisamment robuste pour empêcher un actionnement accidentel ou non souhaité. Or, si la serrure se déverrouille de manière accidentelle, le dispositif est actionné automatiquement et la dose est distribuée, même si l'utilisateur ne l'a pas souhaité.

Les documents WO2004028608, US3456646, US5119806, WO2017176704, NZ562769, US2008156321, WO2008070516, WO2010003846 et WO2013178951 décrivent des dispositifs de l'état de la technique.

La présente invention a pour but de fournir un dispositif de distribution de produit fluide synchronisé avec l'inhalation qui ne reproduit pas les inconvénients susmentionnés.

La présente invention a aussi pour but de fournir un dispositif de distribution de produit fluide synchronisé avec l'inhalation qui améliore la fiabilité de fonctionnement, en garantissant un actionnement efficace à chaque inhalation.

La présente invention a aussi pour but de fournir un dispositif de distribution de produit fluide synchronisé avec l'inhalation qui minimise les risques d'actionnement accidentel ou non souhaité.

La présente invention a également pour but de fournir un dispositif de distribution de produit fluide synchronisé avec l'inhalation qui ne présente pas un seuil de déclenchement trop élevé, permettant ainsi à des personnes relativement faibles, telles que des personnes malades ou âgées, d'utiliser le dispositif de manière sûre et fiable.

La présente invention a également pour but de fournir un dispositif de distribution de produit fluide synchronisé avec l'inhalation qui soit simple et peu coûteux à fabriquer et à assembler.

La présente invention a également pour but de fournir un dispositif de distribution de produit fluide synchronisé avec l'inhalation qui évite les risques de dysfonctionnement de la valve dus à un mauvais remplissage de la chambre de valve après actionnement.

La présente a donc pour objet un dispositif de distribution de produit fluide synchronisé avec l'inhalation, comportant un corps pourvu d'un embout buccal, un réservoir de produit contenant un produit fluide et un gaz propulseur étant monté axialement coulissant par rapport audit corps, une valve doseuse, comportant une soupape, étant assemblée au moyen d'un élément de fixation, telle qu'une capsule sertissable, sur ledit réservoir pour distribuer sélectivement le produit fluide, ledit dispositif comportant:
- un élément de blocage pivotant sur un axe de pivotement B entre une position de blocage, dans laquelle ladite valve doseuse ne peut pas être actionnée, et une position d'actionnement, dans laquelle ladite valve doseuse peut être actionnée,
- un élément déclencheur pivotant sur un axe de pivotement C entre une position de verrouillage, dans laquelle il bloque ledit élément de blocage dans sa position de blocage, et une position de libération, dans laquelle il ne bloque pas ledit élément de blocage, et
- un système de déclenchement commandé par l'inhalation comportant un organe sensible à l'inhalation, déformable et/ou déplaçable sous l'effet de l'inhalation, ledit organe sensible à l'inhalation coopérant avec ledit élément déclencheur, de sorte que lorsque ledit organe sensible à l'inhalation se déforme et/ou se déplace, il déplace et/ou déforme ledit élément déclencheur vers sa position de libération, permettant ainsi le déplacement et/ou la déformation dudit élément de blocage de sa position de blocage vers sa position d'actionnement,
ledit dispositif comportant un organe d'actionnement monté axialement déplaçable, notamment coulissant, dans ledit corps entre une position de repos et une position chargée, un ressort étant disposé entre ledit organe d'actionnement et ledit réservoir ou un élément solidaire dudit réservoir, de sorte que lorsque ledit organe d'actionnement se déplace vers sa position chargée, ledit ressort est comprimé, pour transmettre une force axiale F audit réservoir, ladite force axiale F s'étendant selon un axe Y perpendiculaire audit axe de pivotement B dudit élément de blocage, ledit axe Y étant espacé dudit axe de pivotement B d'une distance d non nulle, ladite distance d étant inférieure à 2,5 mm, avantageusement inférieure à 1 mm, de préférence environ 0,4 mm.

Avantageusement, une bague est fixée, notamment encliquetée, autour dudit élément de fixation, ladite bague comportant une projection axiale coopérant avec ledit élément de blocage, de telle sorte qu'en position de blocage dudit élément de blocage, ladite projection axiale de ladite bague coopère avec une extension de blocage axiale dudit élément de blocage pour ainsi empêcher un déplacement axial dudit réservoir, et en position d'actionnement dudit élément de blocage, ladite projection axiale de ladite bague coopère avec un évidement axial dudit élément de blocage pour ainsi permettre un déplacement axial dudit réservoir.

Avantageusement, en position de blocage dudit élément de blocage, ladite projection axiale de ladite bague sollicite ledit élément de blocage vers sa position d'actionnement.

Avantageusement, une frette est emmanchée autour de ladite bague.

Avantageusement, ledit élément de blocage comporte une projection de verrouillage qui, en position de verrouillage de l'élément déclencheur, coopère avec un épaulement de verrouillage dudit élément déclencheur pour définir une serrure empêchant le déplacement et/ou la déformation dudit élément de blocage hors de sa position de blocage,

Avantageusement, la force F' exercée en position de blocage par ladite projection de verrouillage dudit élément de blocage sur ledit épaulement de verrouillage dudit élément déclencheur s'étend selon un axe Z perpendiculaire audit axe de pivotement C dudit élément déclencheur.

Avantageusement, ledit axe Z est espacé dudit axe de pivotement C d'une distance d' non nulle, ladite distance d' étant inférieure à 2 mm, avantageusement inférieure à 1 mm, de préférence environ 0,25 mm.

Avantageusement, ladite serrure forme, en position de verrouillage de l'élément déclencheur, un premier point de contact entre ledit élément de blocage et ledit élément déclencheur, ledit élément de blocage comportant une projection d'appui qui, en position de verrouillage de l'élément déclencheur, coopère avec une surface d'appui dudit élément déclencheur pour former, en position de verrouillage de l'élément déclencheur, un second point de contact entre ledit élément de blocage et ledit élément déclencheur, ledit second point de contact étant, en position de verrouillage de l'élément déclencheur, à une distance dudit axe C de l'élément déclencheur supérieure à la distance entre ledit axe C et ledit premier point de contact.

Avantageusement, une poignée d'actionnement latérale est montée déplaçable en rotation et en translation sur ledit corps entre une position de repos et une position d'utilisation, un déplacement de ladite poignée d'actionnement latérale vers sa position d'utilisation déplaçant axialement ledit organe d'actionnement vers sa position chargée.

Avantageusement, ladite poignée d'actionnement latérale comporte une première zone d'appui P1 sur ledit organe d'actionnement et une seconde zone d'appui P2 sur le corps.

Avantageusement, ladite première zone d'appui P1 est un point de pivotement et ladite seconde zone d'appui P2 est une surface de glissement radial.

Avantageusement, ledit dispositif comporte un organe bloquant déplaçable et/ou déformable entre une position bloquante et une position non bloquante, ledit organe bloquant, en position bloquante, coopérant avec ledit élément déclencheur pour l'empêcher de se déplacer vers sa position de libération, ladite poignée d'actionnement latérale comportant une projection coopérant avec ledit organe bloquant lorsque ladite poignée d'actionnement latérale est déplacée vers sa position d'utilisation, pour déplacer et/ou déformer ledit organe bloquant vers sa position non bloquante.

Avantageusement, ledit organe sensible à l'inhalation comporte une membrane déformable définissant une chambre d'air déformable, ladite membrane déformable étant fixée audit élément déclencheur, ladite membrane déformable étant déformée lors de l'inhalation de telle sorte qu'elle déplace ledit élément déclencheur de sa position de verrouillage vers sa position de libération.

Avantageusement, ledit embout buccal comporte une ouverture reliée avec l'intérieur du corps, ladite ouverture étant fermée en début d'inhalation par un clapet, de sorte que le flux d'inhalation est initialement principalement transmis au système de déclenchement par l'inhalation.

Avantageusement, ledit clapet est ouvert lorsque ladite bague se déplace axialement ensemble avec ledit réservoir.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique partielle en perspective éclatée d'un dispositif de distribution de produit fluide, selon un mode de réalisation avantageux,
- la figure 2 est une vue schématique en perspective découpée d'une partie du dispositif de la figure 1 après assemblage, en position de repos,
- la figure 3 est une vue schématique en section d'une autre partie du dispositif de la figure 1 après assemblage, en position de repos,
- la figure 4 est une vue schématique en perspective découpée du dispositif de la figure 1 après assemblage, en position de repos,
- la figure 5 est une vue similaire à celle de la figure 4, après actionnement de la poignée d'actionnement,
- la figure 6 est une vue schématique en section du dispositif de la figure 1, en position de repos,
- la figure 7 est une vue similaire à celle de la figure 6, après actionnement de la poignée d'actionnement et avant inhalation,
- la figure 8 est une vue schématique de détail agrandie de la figure 7,
- la figure 9 est une vue schématique en perspective de la bague,
- la figure 10 est une vue schématique en perspective de l'élément déclencheur,
- la figure 11 est une vue schématique en perspective de l'élément de blocage,
- la figure 12 est une vue similaire à celle de la figure 7, en début d'inhalation,
- la figure 13 est une vue schématique de détail agrandie de la figure 12,
- les figures 14 à 17 sont des vues similaires à celle de la figure 13, à différentes étapes d'un cycle d'actionnement,
- la figure 18 est une vue schématique partielle en section de côté du dispositif de la figure 1, en position de repos,
- la figure 19 est une vue schématique partielle en section de face du dispositif de la figure 1, en position de repos,
- la figure 20 est une vue similaire à celle de la figure 18, après actionnement,
- la figure 21 est une vue similaire à celle de la figure 19, après actionnement, et
- les figures 22 à 24 sont des vues schématiques partielles en section du dispositif de la figure 1, respectivement en position de repos, en cours d'actionnement et en cours de retour vers la position de repos.

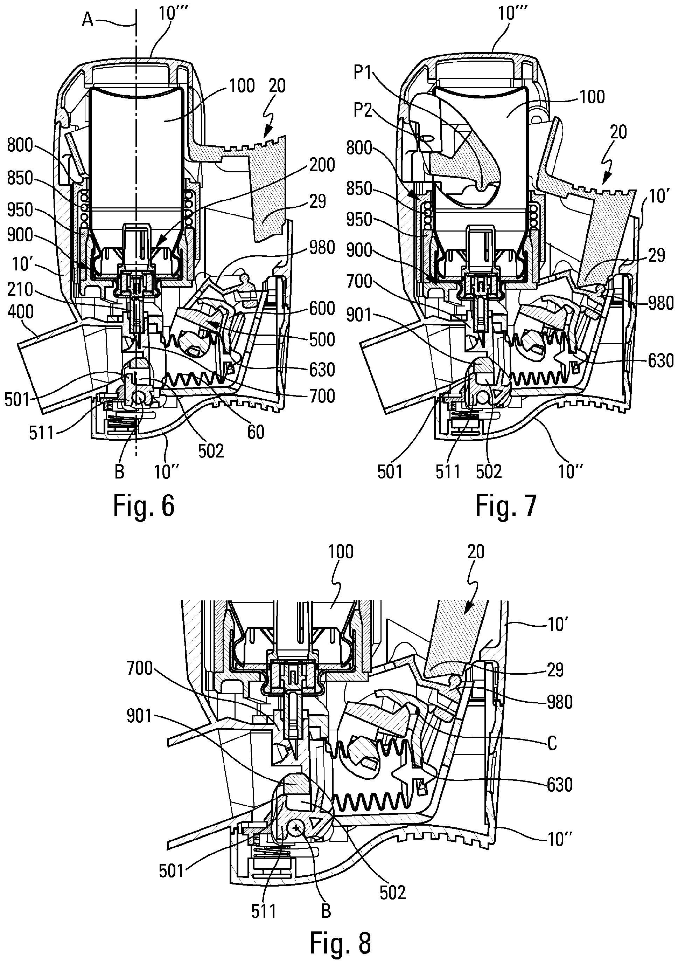

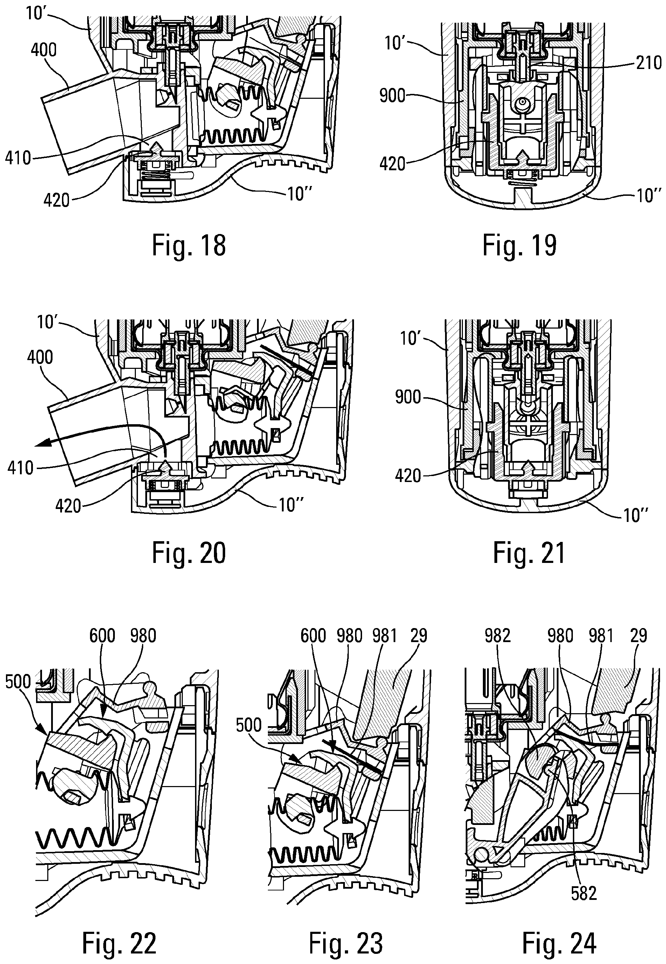

Dans la description, les termes "supérieur", "inférieur", "haut" et "bas" se réfèrent à la position du dispositif représentée notamment sur les figures 6 à 8 et 12 à 24. Les termes "axial" et "radial", sauf si autrement spécifié, se réfèrent à l'axe central vertical A de la valve représenté sur la figure 6. Les termes "proximal" et "distal" se réfèrent à l'embout buccal.

L'invention s'applique plus particulièrement à des dispositifs d'inhalation du type à valve aérosol pour une distribution orale, comme cela sera décrit plus en détail ci-après, mais elle pourrait aussi s'appliquer à d'autres types de dispositifs d'inhalation, par exemple du type nasal.

Les figures représentent des modes de réalisation avantageux de l'invention, mais il est entendu que l'une ou plusieurs des pièces constitutives décrites ci-après pourrai(en)t être réalisée(s) différemment tout en procurant des fonctions similaires voire identiques.

En référence aux dessins, le dispositif comporte un corps 10 pourvu d'un embout buccal 400.

Le corps 10 peut être réalisé en une seule pièce ou en plusieurs pièces assemblées les unes aux autres. Dans les exemples représentés, non limitatifs, le corps 10 comporte trois parties, une partie centrale 10', une partie inférieure 10" et une partie supérieure 10"'. Dans la suite de la description, le corps sera désigné de manière globale par la référence numérique 10.

L'embout buccal 400 définit un orifice de distribution à travers lequel l'utilisateur va inhaler lors de l'utilisation du dispositif. L'embout buccal 400 peut être réalisé d'une pièce avec le corps 10. Dans les exemples représentés sur les dessins, il est formé sur la partie inférieure de corps 10". Un capot de protection amovible (non représenté) peut être prévu sur ledit embout buccal 400, notamment lors des périodes de stockage, que l'utilisateur va retirer avant utilisation.

Le corps 10 contient un réservoir 100, contenant le produit à distribuer et un gaz propulseur, tel qu'un gaz du type HFA, une valve doseuse 200 étant montée sur ledit réservoir 100 pour distribuer sélectivement le produit. La valve doseuse 200 comporte un corps de valve et une soupape 210 axialement déplaçable lors de l'actionnement par rapport audit corps de valve, et donc par rapport audit réservoir 100. Cette valve doseuse 200 peut être d'un type quelconque approprié. Elle est fixée au réservoir 100 par un élément de fixation 5, de préférence une capsule sertie, de préférence avec interposition d'un joint de col.

Avantageusement, lors de l'actionnement, la soupape 210 est fixe par rapport au corps 10, et c'est le réservoir 100 qui est déplacé axialement par rapport au corps 10 entre une position distale, qui est la position de repos, et une position proximale, qui est la position d'actionnement.

L'orifice de sortie de la soupape 210 de ladite valve doseuse 200 est relié via un canal audit embout buccal 400, à travers lequel l'utilisateur inhale le produit distribué. De manière connue, ladite soupape 210 est reçue dans un puits de soupape 700 qui définit au moins partiellement ledit canal.

Le dispositif comporte une bague 900 qui est avantageusement fixée autour dudit élément de fixation 5, par exemple par encliquetage au moyen de pattes d'encliquetage 905. Avantageusement, une frette 950 est emmanchée autour de ladite bague 900, pour maintenir lesdites pattes d'encliquetage 905 en position encliquetée. La bague 900 comporte une projection axiale 901 dont la fonction sera décrite ci-après.

Un organe d'actionnement 800 est avantageusement assemblé autour du réservoir 100. Cet organe d'actionnement 800 comporte un manchon creux 801 disposé dans le corps 10 autour du réservoir 100, avec un ressort 850 disposé entre le fond dudit manchon creux 801 et le réservoir 100 ou un élément solidaire dudit réservoir 100, tel que la bague 900 ou la frette 950. Le manchon creux 801 est déplaçable axialement, notamment coulissant, par rapport audit réservoir 100 entre une position de repos et une position chargée. Ainsi, lorsque l'utilisateur souhaite actionner la valve doseuse 200, il appuie sur ledit organe d'actionnement 800. Ceci va déplacer axialement ledit manchon creux 801 vers sa position chargée et ainsi comprimer ledit ressort 850, qui va donc transmettre une force axiale F audit réservoir 100, notamment via ladite frette 950 dans l'exemple représenté. Cette force axiale F est sensiblement la même à chaque actionnement. Tant que l'utilisateur maintient son appui sur ledit organe d'actionnement 800, ledit ressort 850 est comprimé et sollicite axialement ledit réservoir 100 vers sa position d'actionnement.

Une poignée d'actionnement latérale 20 est avantageusement montée déplaçable en rotation et en translation sur le corps 10. Cette poignée 20, lorsque déplacée de sa position de repos, visible notamment sur la figure 6, vers sa position d'utilisation, visible notamment sur la figure 7, déplace axialement ledit organe d'actionnement 800 pour comprimer le ressort 850.

La poignée d'actionnement latérale 20 comporte avantageusement une première zone d'appui P1 sur ledit organe d'actionnement 800 et une seconde zone d'appui P2 sur le corps 10.

Dans le mode de réalisation représenté sur les figures, la première zone d'appui P1 est un point de pivotement et la seconde zone d'appui P2 est une surface de glissement radial. Lors de l'actionnement de la poignée 20, le point de pivotement P1 va coulisser axialement vers le bas par rapport au corps 10, alors que le point de contact dans la zone P2 va se déplacer radialement vers l'intérieur par rapport au corps 10.

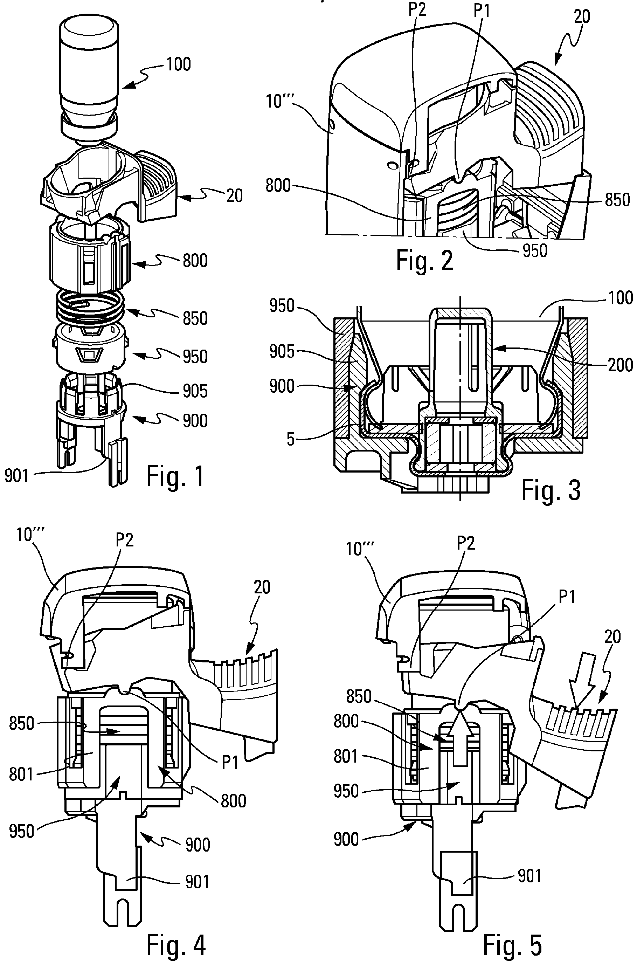

Dans cette mise en œuvre, la poignée 20 est donc déplaçable à la fois en rotation et en translation. Ceci permet de démultiplier l'effort de l'utilisateur, tout en gardant un encombrement réduit. Cette démultiplication permet d'actionner la valve 200 avec un effort bien inférieur à celui qui serait nécessaire si l'on devait appuyer axialement sur le fond du réservoir 100. En particulier, dans l'exemple représenté et particulièrement bien visible sur les figures 4 et 5, l'effort nécessaire pour actionner axialement la valve 200 est typiquement de 40-45N (lié à la raideur du ressort 850) alors que l'effort pour actionner la poignée d'actionnement latérale 20 est seulement d'environ 15N. La démultiplication est donc ici d'environ un facteur trois. Il est possible d'augmenter encore ce ratio, notamment en jouant sur la géométrie des différentes pièces.

Tant que l'utilisateur maintient son appui sur ladite poignée, ledit ressort 850 est comprimé et sollicite axialement ledit réservoir 100 vers sa position d'actionnement.

Après chaque actionnement, lorsque l'utilisateur relâche sa pression sur la poignée 20, ce qui se fait naturellement, celle-ci revient automatiquement vers sa position de repos sous l'effet du ressort 850. Ceci permet d'éviter le risque qu'après actionnement de la valve doseuse 200, cette dernière reste en position actionnée, ce qui pourrait avoir pour conséquence que la chambre de la valve se remplit d'air et que la dose suivante est incomplète voire que la valve fuit. Ceci est une des problématiques rencontrées actuellement par les dispositifs existants sur le marché.

Le dispositif comporte un élément de blocage 500 déplaçable et/ou déformable entre une position de blocage, dans laquelle ladite valve doseuse 200 ne peut pas être actionnée, et une position d'actionnement, dans laquelle ladite valve doseuse 200 peut être actionnée. En position de repos, ledit élément de blocage 500 est en position de blocage, et c'est l'inhalation de l'utilisateur à travers l'embout buccal 400 qui déplace et/ou déforme ledit élément de blocage 500 vers sa position d'actionnement. En d'autres termes, tant que l'utilisateur n'a pas inhalé, l'actionnement de la valve doseuse 200 est impossible, et ce n'est que lorsqu'il inhale que ladite valve doseuse 200 peut être actionnée, par déplacement axial du réservoir 100 dans le corps 10.

Comme cela sera décrit plus en détails ci-après, l'élément de blocage 500, en position de blocage, empêche le déplacement axial du réservoir 100 dans le corps 10. Lors de l'inhalation, cet élément de blocage 500 est déplacé et/ou déformé de telle sorte qu'il ne bloque plus le déplacement axial du réservoir 100 dans le corps 10. Ainsi, après inhalation, un tel déplacement axial du réservoir 100 provoque l'actionnement de la valve doseuse 200 et la distribution d'une dose de produit synchronisée avec cette inhalation.

Ainsi, en l'absence d'inhalation, il n'y a aucun risque qu'une dose de produit actif soit perdue par un actionnement malencontreux ou incomplet dans lequel l'utilisateur n'exercerait pas d'inhalation. L'actionnement de la valve 200 et l'expulsion d'une dose de produit fluide ne sont donc possibles que si l'utilisateur inhale et que simultanément il actionne la poignée d'actionnement 20. En variante, on pourrait aussi envisager que l'utilisateur appuie axialement directement sur le fond du réservoir, ou alors utiliser un système d'actionnement automatique, qui appliquerait un appui axial sur le réservoir indépendamment de l'utilisateur.

Le dispositif comporte un système de déclenchement commandé par l'inhalation de l'utilisateur, qui est destiné à déplacer et/ou déformer ledit élément de blocage 500 de sa position de blocage vers sa position d'actionnement, lorsque l'utilisateur inhale à travers l'embout buccal 400.

Ce système de déclenchement comporte un organe sensible à l'inhalation 60 déformable et/ou déplaçable sous l'effet de l'inhalation, cet organe sensible à l'inhalation 60 étant adapté, lorsqu'il se déforme et/ou se déplace, à permettre le déplacement et/ou la déformation dudit élément de blocage 500 de sa position de blocage vers sa position d'actionnement.

Comme cela sera décrit plus en détails ci-après, l'organe sensible à l'inhalation peut être réalisé sous la forme d'une chambre d'air déformable 60, par exemple un soufflet ou une poche déformable.

De cette manière, le système de déclenchement commandé par l'inhalation n'est pas situé dans le flux de l'aspiration de l'utilisateur mais est formé par une chambre spécifique, à savoir la chambre d'air 60. Ceci diffère des systèmes fonctionnant à l'aide d'un volet qui se déplace/déforme dans le flux d'aspiration, dans lesquels, après déclenchement, l'utilisateur va aspirer l'air qui se trouve de chaque côté du volet. Ici, le système fonctionne en dépression et l'utilisateur n'aspire que le petit volume d'air qui était à l'intérieur de la chambre d'air 60 avant sa déformation. Le système selon l'invention est ainsi beaucoup plus stable et performant.

L'élément de blocage 500 est avantageusement monté pivotant autour d'un axe B sur le corps 10 entre une position de blocage et une position d'actionnement. Dans l'exemple représenté, ledit axe B peut être formé par des projections prévues sur une surface inférieure du corps 10, l'élément de blocage 500 comportant des profils complémentaires 511 adaptés à pivoter sur lesdites projections. D'autres mises en œuvre sont aussi possibles.

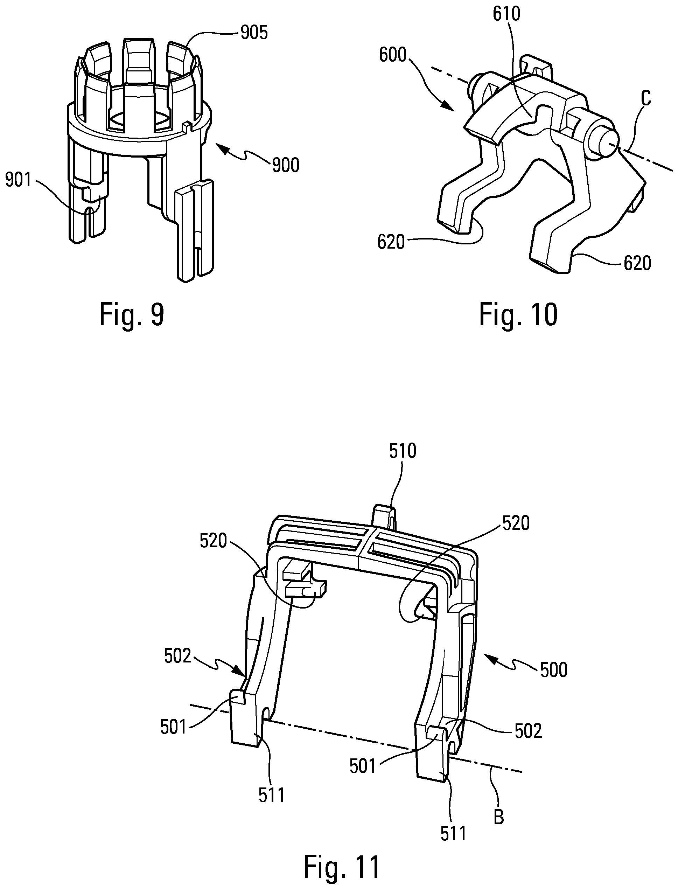

L'élément de blocage 500 comporte au moins une, de préférence deux extensions de blocage 501, qui coopère chacune en position de blocage avec une projection axiale 901 de ladite bague 900 solidaire du réservoir 100. La figure 11 représente une vue en perspective de cet élément de blocage 500.

Lorsque l'élément de blocage 500 se déplace vers sa position d'actionnement, notamment par pivotement autour de l'axe B, chaque extension de blocage 501 se déplace hors de contact avec la projection axiale 901 respective. En particulier, ledit élément de blocage 500 comporte de manière adjacente à chaque extension de blocage 501 un évidement axial 502 dans lequel la projection axiale 901 respective peut coulisser axialement, pour ainsi permettre un coulissement axial dudit réservoir 100 dans ledit corps 10, provoquant l'actionnement de la valve 200 et la distribution d'une dose de produit fluide.

L'élément de blocage 500 est retenu en position de blocage par un élément déclencheur 600. La figure 10 représente une vue en perspective de cet élément déclencheur 600. Cet élément déclencheur 600 est avantageusement monté pivotant autour d'un axe C sur le corps 10, entre une position de verrouillage, dans laquelle il bloque ledit élément de blocage 500 dans sa position de blocage, et une position de libération, dans laquelle il ne bloque plus ledit élément de blocage 500.

Avantageusement, les axes B et C sont parallèles.

L'élément de blocage 500 et l'élément déclencheur 600 définissent ensemble une serrure. En particulier, ledit élément déclencheur 600 comporte un épaulement de verrouillage 610 qui, en position de verrouillage, coopère avec une projection de verrouillage 510 de l'élément de blocage 500, empêchant le pivotement dudit élément de blocage 500 hors de sa position de blocage. Ainsi, lorsque ledit élément déclencheur 600 est en position de verrouillage, il empêche le déplacement de l'élément de blocage 500 vers sa position d'actionnement, ce qui bloque le déplacement axial du réservoir 100 et donc l'actionnement de la valve doseuse 200.

Le système de blocage de la présente invention comporte donc deux étages : un premier étage formé par la serrure entre l'élément de blocage 500 et l'élément déclencheur 600, et un second étage formé par le blocage entre l'élément de blocage 500 et le réservoir 100, via la bague 900.

Ce système de blocage permet le déverrouillage d'un gros effort (typiquement environ 40-45N) par un petit effort généré par l'inhalation. L'élément de blocage 500 arrête la translation du réservoir 100 lorsqu'il est soumis à un effort F (de 45N par exemple) par l'appui par l'utilisateur sur l'organe d'actionnement 800, de préférence via la poignée d'actionnement 20. Cet élément de blocage 500 interagit avec l'élément déclencheur 600 et est bloqué/libéré par celui-ci. Le déplacement dudit élément déclencheur 600 est commandé par l'inhalation.

La géométrie du système de blocage permet une amplification (effort déverrouillé/effort déverrouillant) très importante, typiquement de l'ordre de 100.

L'élément de blocage 500 et l'élément déclencheur 600 ont de préférence deux points de contact géométriquement distant:
- un premier point de contact, formé par la serrure définie entre l'épaulement de verrouillage 610 et la projection 510, se trouvant avantageusement proche de l'axe de pivotement C de l'élément déclencheur 600;
- un second point de contact éloigné du premier point de contact, formé par la coopération d'une projection latérale 520 de l'élément de blocage 500 avec une surface d'appui 620 de l'élément déclencheur 600; avantageusement, ce second point de contact est, en position de verrouillage, à une distance de l'axe C de l'élément déclencheur 600 supérieure à la distance entre ledit axe C et le premier point de contact; avantageusement, ce second point de contact est le premier contact qui se rompt lors de l'actionnement du dispositif, dès que l'utilisateur commence à inhaler.

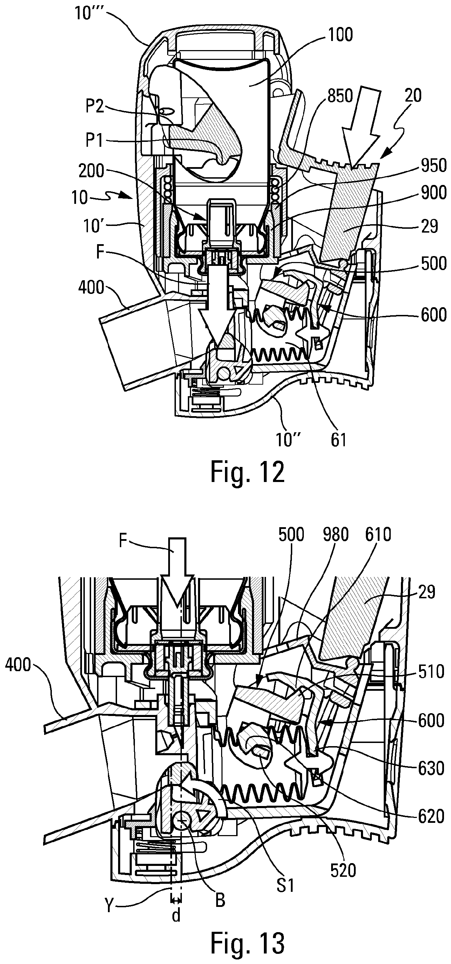

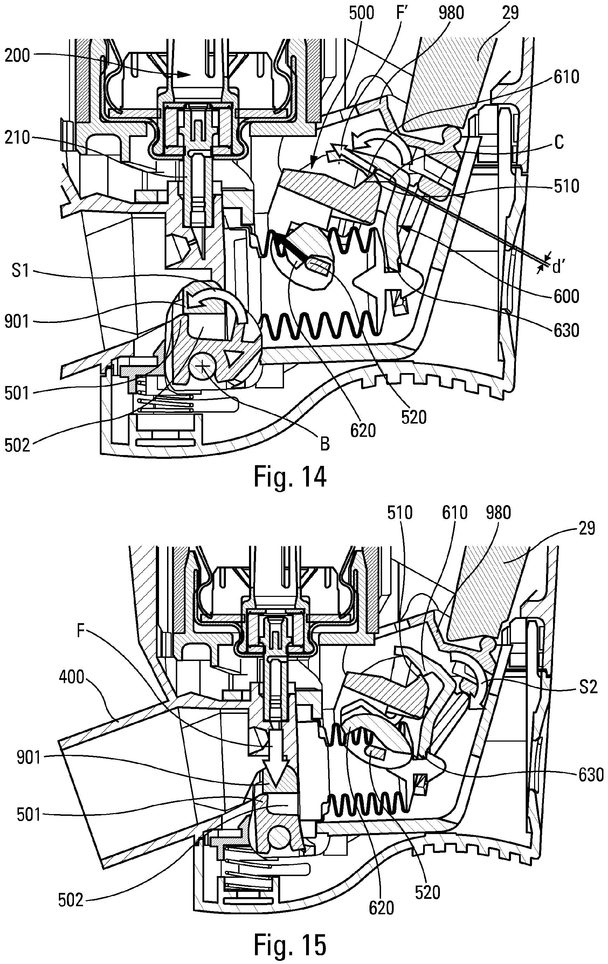

En position de blocage, la force F générée par l'appui axial de l'organe d'actionnement 800 sur la frette 950 solidaire du réservoir 100 est appliquée par les projections axiales 901 de la bague 900 sur l'élément de blocage 500 au niveau des extensions 501, avec pour effet de solliciter ledit élément de blocage en rotation dans un sens S1, qui renforce la position fermée de la serrure et la rend stable. Les figures 13 et 14 illustrent notamment cette position de blocage.

La force de déverrouillage générée par l'inhalation est appliquée sur l'élément déclencheur 600 par la membrane déformable 61, de préférence en un point 630 éloigné de l'axe du pivot C; cette force de déverrouillage vise à faire tourner ledit élément déclencheur 600 dans le sens S2 contraire au sens S1, comme illustré sur la figure 15.

Le couple que subit l'élément de blocage 500 est maitrisé par la distance entre l'axe d'effort sur lequel la force F est appliquée sur les extensions de blocage 501 de l'élément de blocage et l'axe de pivot B dudit élément de blocage 500. On cherche à avoir une distance d la plus faible possible afin d'avoir le couple le plus faible possible. Cette distance d, visible sur la figure 13, est non nulle, et est inférieure à 2 mm, avantageusement inférieure à 1 mm, de préférence environ 0,4 mm.

Le couple que subit l'élément déclencheur 600 est maitrisé par la distance entre l'axe d'effort portant la force F' que subit l'élément déclencheur 600 de la part de l'élément de blocage 500 et l'axe de pivot C dudit élément déclencheur 600. Ici aussi, on cherche à avoir une distance d' la plus faible possible afin d'avoir le couple le plus faible possible. Cette distance d', visible sur la figure 14, est non nulle, et est inférieure à 2 mm, avantageusement inférieure à 1 mm, de préférence environ 0,25 mm.

Grâce à ce système d'effort de la serrure, l'effort nécessaire pour faire pivoter l'élément déclencheur 600 est très faible et peut être généré par la membrane déformable 61, qui permet de transformer la dépression générée par l'inhalation en force de déverrouillage.

Avantageusement, l'embout buccal 400 comporte une ouverture 410 reliée avec l'intérieur du corps 10. Cette ouverture 410 est fermée au repos et en début d'inhalation par un clapet 420, de sorte que le flux d'inhalation est initialement principalement transmis au système de déclenchement par l'inhalation, dans cet exemple la chambre d'air déformable 60. Ceci permet d'optimiser ce déclenchement par l'inhalation. Lorsque l'élément de blocage 500 se déplace vers sa position d'actionnement sous l'effet de l'inhalation, et donc le réservoir 100 se déplace axialement par rapport au corps 10 pour actionner la valve doseuse 200 pour distribuer une dose de produit fluide, ladite bague 900, solidaire du réservoir 100, déplace ledit clapet 420 vers une position ouverte. Lorsque lesdites ouvertures 410 sont ainsi ouvertes, en cours d'actionnement, un appel d'air est généré, ce qui permet d'augmenter le flux d'inhalation. Ceci optimise la synchronisation entre la distribution de la dose et l'inhalation de l'utilisateur, et favorise une bonne distribution de la dose dans les poumons de l'utilisateur.

Avantageusement, l'élément déclencheur 600 peut être accessible de l'extérieur du corps 10 et/ou de la partie inférieure de corps 10'. Ceci permet en cas de besoin de déplacer manuellement l'élément déclencheur 600, pour pouvoir réaliser un actionnement de la valve doseuse 200 même sans inhalation, par exemple si la personne devant recevoir la dose de produit fluide est incapable de réaliser une inhalation suffisante. Il s'agit donc d'une sécurité.

Dans les modes de réalisation représentés sur les figures, l'organe sensible à l'inhalation 60 est réalisé sous la forme d'une chambre d'air déformable. Avantageusement, cette chambre d'air comprend une membrane déformable 61 qui est reliée d'une part à ladite partie inférieure de corps 10' et d'autre part audit élément déclencheur 600. Avantageusement, comme visible sur les figures, la membrane 61 a une forme de soufflet et forme une chambre sensiblement étanche. D'autres formes sont possibles, notamment une simple poche ou diaphragme. Un plot peut fixer ladite membrane 61 à un orifice ou bord 630 dudit élément déclencheur 600.

Lors de l'inhalation, la membrane déformable 61 se déforme et/ou se contracte sous l'effet de la dépression générée par l'inhalation, provoquant le déplacement de l'élément déclencheur 600 de sa position de verrouillage vers sa position de libération. Ceci permet d'ouvrir la serrure définie entre l'élément de blocage 500 et l'élément déclencheur 600, et donc le déplacement dudit élément de blocage 500 de sa position de blocage vers sa position d'actionnement.

La valve 200 n'est donc actionnée qu'au moment de l'inhalation, de sorte que la dose de produit fluide est expulsée hors de l'orifice de distribution simultanément à l'inhalation.

Avantageusement, le dispositif comporte un organe bloquant 980 déplaçable et/ou déformable entre une position bloquante et une position non bloquante. En position bloquante, cet organe bloquant 980 coopère avec l'élément déclencheur 600 pour l'empêcher de se déplacer vers sa position de libération. La poignée d'actionnement latérale 20 comporte avantageusement une projection 29 coopérant avec ledit organe bloquant 980 lorsque ladite poignée d'actionnement latérale 20 est déplacée vers sa position d'utilisation. Ceci déplace et/ou déforme ledit organe bloquant 980 vers sa position non bloquante.

Ainsi, si l'utilisateur inhale sans avoir exercé l'appui axial sur le réservoir 100, la serrure ne sera pas débloquée, puisque l'élément déclencheur 600 ne peut pas pivoter. La chambre d'air 60 étant sensiblement étanche, et le clapet 420 étant fermé sur l'ouverture 410, l'utilisateur se rend compte très vite qu'il ne peut inhaler correctement à travers l'embout buccal, ce qui lui rappelle qu'il faut d'abord actionner la poignée avant d'inhaler. Lorsque l'utilisateur appuie sur la poignée 20, il déplace l'organe bloquant 980 dans sa position non bloquante. Une inhalation provoquera alors un pivotement de l'élément déclencheur 600, et donc l'actionnement du dispositif, comme expliqué précédemment.

Avantageusement, l'organe bloquant 980 comporte un élément élastique (non représenté), tel qu'un ressort de torsion, qui sollicite élastiquement ledit organe bloquant 980 vers sa position bloquante, de sorte que lorsque l'utilisateur relâche la poignée d'actionnement 20, ledit organe bloquant 980 revient automatiquement dans sa position bloquante. Avantageusement, ceci ramène également l'élément déclencheur 600 dans sa position de verrouillage, par exemple via une lame flexible 981 appropriée.

L'organe bloquant 980 comporte en outre avantageusement une extension de réarmement 982 qui coopère avec une partie de réarmement 582 de l'élément de blocage 500, de sorte que lorsque l'organe bloquant 980 revient dans sa position bloquante, il ramène l'élément de blocage 500 dans sa position de blocage.

Lorsque l'utilisateur souhaite utiliser le dispositif, il place l'embout buccal 400 dans sa bouche, et exerce manuellement un appui axial sur la poignée d'actionnement 20. Le réservoir 100 est bloqué et empêché de coulisser axialement dans le corps 10 par les extensions de blocage 501 de l'élément de blocage 500 qui bloquent axialement les projections axiales 901 de la bague 900. Parallèlement, le blocage de l'élément déclencheur 600 est supprimé par le déplacement de l'organe bloquant 980, comme visible notamment sur les figures 6 et 7.

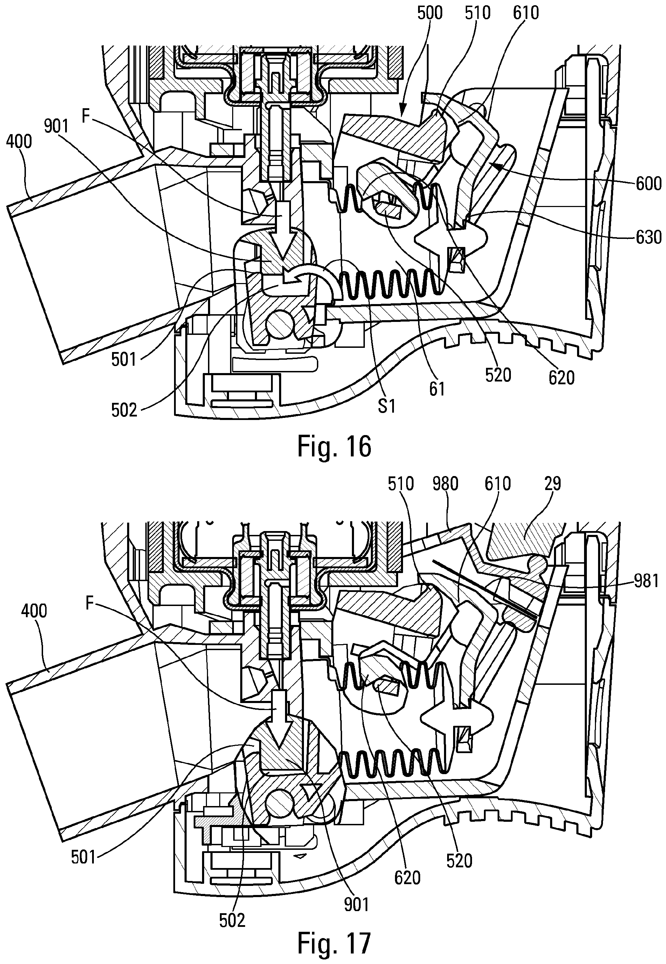

Lorsque l'utilisateur inhale à travers l'embout buccal 400, il va déformer la membrane déformable 61, ce qui va faire pivoter l'élément déclencheur 600 fixé à ladite membrane déformable 61. Ce déplacement de l'élément déclencheur 600 va libérer la serrure formée entre l'épaulement de verrouillage 610 de l'élément déclencheur 600 et la projection 510 de l'élément de blocage 500, comme visible sur les figures 14 à 17. Sous l'effet de la force axiale F transmise par le réservoir 100, l'élément de blocage 500 va pivoter permettant le coulissement axial du réservoir 100 dans le corps 10 vers sa position de distribution, et donc l'actionnement de la valve 200. Parallèlement, la bague 900va ouvrir le clapet 420. Cette position de distribution est représentée sur les figures 17, 20 et 21.

En fin d'inhalation, lorsque l'utilisateur relâche sa pression sur le fond du réservoir 100, notamment en relâchant sa pression sur la poignée 20, ledit réservoir 100 remonte axialement dans le corps 10 en direction de sa position de repos sous l'effet du ressort de rappel de la valve 200, et simultanément la soupape 210 de la valve doseuse revient en position de repos, en chargeant une nouvelle dose de produit fluide dans la chambre de valve. L'élément déclencheur 600 est ramené dans sa position initiale, notamment par l'élasticité de la membrane 61 et/ou la lame élastique 981 de l'organe bloquant 980. L'élément de blocage 500 revient dans sa position de blocage, via l'extension de réarmement 982 de l'organe bloquant 980.

Le dispositif est alors prêt pour une autre utilisation.

Il est à noter que le dispositif pourrait comporter un compteur de doses électronique, avantageusement assemblé dans le corps ou dans la poignée. Ce compteur peut notamment détecter les déplacements du réservoir. En variante, le compteur pourrait être relié à un capteur, notamment un capteur à membrane, qui détecte la distribution de la dose de produit fluide, par exemple dans le puits de soupape. D'autres moyens d'actionner un tel compteur électronique sont aussi possibles, par exemple la détection du déplacement de la soupape de la valve doseuse par rapport au corps de valve.

Le dispositif peut alors aussi comporter des moyens d'émission de signaux pour communiquer à distance notamment des informations relatives aux actionnements du dispositif. En particulier, le corps et/ou la poignée peut comporter un module d'émission de signaux, pour communiquer à distance avec une base quelconque. Des moyens d'alimentation appropriés sont avantageusement prévus.

Avantageusement, le module électronique peut comprendre notamment une carte comportant un commutateur électrique qui envoie une impulsion. Le module peut également comporter un afficheur et/ou utiliser une connexion Bluetooth ou Wifi pour envoyer les informations sur un périphérique annexe. Des capteurs appropriés peuvent être prévus, tel que des capteurs de débit et/ou de pression, pour détecter divers paramètres du flux d'inhalation.

Le commutateur peut être actionné grâce au mouvement de l'élément de blocage.

Associés à un compteur de dose qui compte chaque dose effectivement émise et au dispositif de synchronisation avec l'inhalation de l'invention, ces moyens d'émission de signaux permettent de transmettre de manière absolument fiable chaque distribution de dose, par exemple à un médecin ou toute autre personne souhaitant surveiller l'utilisation du dispositif d'inhalation par l'utilisateur. Le dispositif de synchronisation avec l'inhalation garantit que l'utilisateur inhale à chaque fois qu'il actionne le dispositif, et le compteur enregistre chaque dose distribuée, ainsi que différents paramètres associés, tel que l'horodatage de chaque distribution. Le médecin peut ainsi connaitre très exactement les conditions d'utilisation du dispositif par l'utilisateur.

La présente invention est notamment applicable pour le traitement des crises d'asthme ou de BPCO (Broncho Pneumopathie Obstructive) en utilisation avec des formulations de type salbutamol, aclidinium, formoterol, tiotropium, budesonide, fluticasone, indacaterol, glycopyrronium, salmeterol, umeclidinium bromide, vilanterol, olodaterol, striverdi, ou toute combinaison de ces formulations.

La présente invention a été décrite en référence à des modes de réalisation et variantes avantageux, mais il est entendu qu'un homme du métier peut y apporter toutes modifications, sans sortir du cadre de la présente invention tel que défini par les revendications annexées.

## Revendications

1. Dispositif de distribution de produit fluide synchronisé avec l'inhalation, comportant un corps (10) pourvu d'un embout buccal (400), un réservoir de produit (100) contenant un produit fluide et un gaz propulseur étant monté axialement coulissant par rapport audit corps (10), une valve doseuse (200), comportant une soupape (210), étant assemblée au moyen d'un élément de fixation (5), telle qu'une capsule sertissable, sur ledit réservoir (100) pour distribuer sélectivement le produit fluide, ledit dispositif comportant:
- un élément de blocage (500) pivotant sur un axe de pivotement B entre une position de blocage, dans laquelle ladite valve doseuse (200) ne peut pas être actionnée, et une position d'actionnement, dans laquelle ladite valve doseuse (200) peut être actionnée,
- un élément déclencheur (600) pivotant sur un axe de pivotement C entre une position de verrouillage, dans laquelle il bloque ledit élément de blocage (500) dans sa position de blocage, et une position de libération, dans laquelle il ne bloque pas ledit élément de blocage (500), et
- un système de déclenchement commandé par l'inhalation comportant un organe sensible à l'inhalation (60, 61), déformable et/ou déplaçable sous l'effet de l'inhalation, ledit organe sensible à l'inhalation (60, 61) coopérant avec ledit élément déclencheur (600), de sorte que lorsque ledit organe sensible à l'inhalation (60, 61) se déforme et/ou se déplace, il déplace et/ou déforme ledit élément déclencheur (600) vers sa position de libération, permettant ainsi le déplacement et/ou la déformation dudit élément de blocage (500) de sa position de blocage vers sa position d'actionnement,
ledit dispositif comportant un organe d'actionnement (800) monté axialement déplaçable, notamment coulissant, dans ledit corps (10) entre une position de repos et une position chargée, et un ressort (850) **caractérisé en ce que** ledit ressort est disposé entre ledit organe d'actionnement (800) et ledit réservoir (100) ou un élément (900 ; 950) solidaire dudit réservoir (100), de sorte que lorsque ledit organe d'actionnement (800) se déplace vers sa position chargée, ledit ressort (850) est comprimé, pour transmettre une force axiale (F) audit réservoir (100), ladite force axiale (F) s'étendant selon un axe Y perpendiculaire audit axe de pivotement B dudit élément de blocage (500), ledit axe Y étant espacé dudit axe de pivotement B d'une distance d non nulle, ladite distance d étant inférieure à 2,5 mm, avantageusement inférieure à 1 mm, de préférence environ 0,4 mm.

2. Dispositif selon la revendication 1, dans lequel une bague (900) est fixée, notamment encliquetée, autour dudit élément de fixation (5), ladite bague (900) comportant une projection axiale (901) coopérant avec ledit élément de blocage (500), de telle sorte qu'en position de blocage dudit élément de blocage (500), ladite projection axiale (901) de ladite bague (900) coopère avec une extension de blocage axiale (501) dudit élément de blocage (500) pour ainsi empêcher un déplacement axial dudit réservoir (100), et en position d'actionnement dudit élément de blocage (500), ladite projection axiale (901) de ladite bague (900) coopère avec un évidement axial (502) dudit élément de blocage (500) pour ainsi permettre un déplacement axial dudit réservoir (100).

3. Dispositif selon la revendication 2, dans lequel, en position de blocage dudit élément de blocage (500), ladite projection axiale (901) de ladite bague (900) sollicite ledit élément de blocage (500) vers sa position d'actionnement.

4. Dispositif selon la revendication 2 ou 3, dans lequel une frette (950) est emmanchée autour de ladite bague (900).

5. Dispositif selon l'une quelconque des revendications précédentes, dans lequel ledit élément de blocage (500) comporte une projection de verrouillage (510) qui, en position de verrouillage de l'élément déclencheur (600), coopère avec un épaulement de verrouillage (610) dudit élément déclencheur (600) pour définir une serrure empêchant le déplacement et/ou la déformation dudit élément de blocage (500) hors de sa position de blocage,

6. Dispositif selon la revendication 5, dans lequel la force (F') exercée en position de blocage par ladite projection de verrouillage (510) dudit élément de blocage (500) sur ledit épaulement de verrouillage (610) dudit élément déclencheur (600) s'étend selon un axe Z perpendiculaire audit axe de pivotement C dudit élément déclencheur (600).

7. Dispositif selon la revendication 6, dans lequel ledit axe Z est espacé dudit axe de pivotement C d'une distance d' non nulle, ladite distance d' étant inférieure à 2 mm, avantageusement inférieure à 1 mm, de préférence environ 0,25 mm.

8. Dispositif selon l'une quelconque des revendications précédentes, dans lequel ladite serrure forme, en position de verrouillage de l'élément déclencheur (600), un premier point de contact entre ledit élément de blocage (500) et ledit élément déclencheur (600), ledit élément de blocage (500) comportant une projection d'appui (520) qui, en position de verrouillage de l'élément déclencheur (600), coopère avec une surface d'appui (620) dudit élément déclencheur (600) pour former, en position de verrouillage de l'élément déclencheur (600), un second point de contact entre ledit élément de blocage (500) et ledit élément déclencheur (600), ledit second point de contact étant, en position de verrouillage de l'élément déclencheur (600), à une distance dudit axe C de l'élément déclencheur (600) supérieure à la distance entre ledit axe C et ledit premier point de contact.

9. Dispositif selon l'une quelconque des revendications précédentes, dans lequel une poignée d'actionnement latérale (20) est montée déplaçable en rotation et en translation sur ledit corps (10) entre une position de repos et une position d'utilisation, un déplacement de ladite poignée d'actionnement latérale (20) vers sa position d'utilisation déplaçant axialement ledit organe d'actionnement (800) vers sa position chargée.

10. Dispositif selon la revendication 9, dans lequel ladite poignée d'actionnement latérale (20) comporte une première zone d'appui (P1) sur ledit organe d'actionnement (800) et une seconde zone d'appui (P2) sur le corps (10).

11. Dispositif selon la revendication 10, dans lequel ladite première zone d'appui (P1) est un point de pivotement et ladite seconde zone d'appui (P2) est une surface de glissement radial.

12. Dispositif selon l'une quelconque des revendications 9 à 11, comportant un organe bloquant (980) déplaçable et/ou déformable entre une position bloquante et une position non bloquante, ledit organe bloquant (980), en position bloquante, coopérant avec ledit élément déclencheur (600) pour l'empêcher de se déplacer vers sa position de libération, ladite poignée d'actionnement latérale (20) comportant une projection (29) coopérant avec ledit organe bloquant (980) lorsque ladite poignée d'actionnement latérale (20) est déplacée vers sa position d'utilisation, pour déplacer et/ou déformer ledit organe bloquant (980) vers sa position non bloquante.

13. Dispositif selon l'une quelconque des revendications précédentes, dans lequel ledit organe sensible à l'inhalation (60, 61) comporte une membrane déformable (61) définissant une chambre d'air déformable (60), ladite membrane déformable (61) étant fixée audit élément déclencheur (600), ladite membrane déformable (61) étant déformée lors de l'inhalation de telle sorte qu'elle déplace ledit élément déclencheur (600) de sa position de verrouillage vers sa position de libération.

14. Dispositif selon l'une quelconque des revendications précédentes, dans lequel ledit embout buccal (400) comporte une ouverture (410) reliée avec l'intérieur du corps (10), ladite ouverture (410) étant fermée en début d'inhalation par un clapet (420), de sorte que le flux d'inhalation est initialement principalement transmis au système de déclenchement par l'inhalation.

15. Dispositif selon la revendication 14, dans lequel ledit clapet (420) est ouvert lorsque ladite bague (900) se déplace axialement ensemble avec ledit réservoir (100).

## Patentansprüche

1. Fluidprodukt-Verteilungsvorrichtung, die mit der Einatmung synchronisiert ist und einen mit einem Mundstück (400) versehenen Körper (10) aufweist, wobei ein Produktvorratsbehälter (100), der ein Fluidprodukt und ein Gastreibmittel enthält, in Bezug auf den Körper (10) axial gleitend montiert ist, wobei ein Dosierventil (200), das ein Ventilelement (210) aufweist, mittels eines Befestigungselements (5) wie etwa einer crimbbaren Hülse an den Vorratsbehälter (100) angefügt ist, um das Fluidprodukt wahlweise zu verteilen, wobei die Vorrichtung Folgendes umfasst:
- ein Blockierelement (500), das sich um eine Drehachse B zwischen einer Blockierposition, in der das Dosierventil (200) nicht betätigt werden kann, und einer Betätigungsposition, in der das Dosierventil (200) betätigt werden kann, dreht,
- ein Auslöserelement (600), das sich um eine Drehachse C zwischen einer Verriegelungsposition, in der es das Blockierelement (500) in seiner Blockierposition blockiert, und einer Freigabeposition, in der es das Blockierelement (500) nicht blockiert, dreht, und
- ein Auslösesystem, das durch die Einatmung gesteuert wird und ein auf die Einatmung ansprechendes Organ (60, 61) aufweist, das unter der Wirkung der Einatmung verformbar und/oder verlagerbar ist, wobei das auf die Einatmung ansprechende Organ (60, 61) mit dem Auslöserelement (600) zusammenwirkt, derart, dass dann, wenn sich das auf die Einatmung ansprechende Organ (60, 61) verformt und/oder verlagert, es das Auslöserelement (600) in seine Freigabeposition verlagert und/oder verformt, was die Verlagerung und/oder Verformung des Blockierelements (500) aus seiner Blockierposition in seine Betätigungsposition ermöglicht,
wobei die Vorrichtung ein Betätigungsorgan (800), das axial verlagerbar, insbesondere gleitend zwischen einer Ruheposition und einer gespannten Position in dem Körper (10) montiert ist, und eine Feder (850) aufweist, **dadurch gekennzeichnet, dass** die Feder zwischen dem Betätigungsorgan (800) und dem Vorratsbehälter (100) oder einem mit dem Vorratsbehälter (100) fest verbundenen Element (900; 950) angeordnet ist, derart, dass dann, wenn sich das Betätigungsorgan (800) in seine gespannte Position verlagert, die Feder (850) komprimiert wird, um eine axiale Kraft (F) an den Vorratsbehälter (100) zu übertragen, wobei die axiale Kraft (F) längs einer Achse Y senkrecht zu der Drehachse B des Blockierelements (500) orientiert ist, wobei die Achse Y von der Drehachse B um einen von null verschiedenen Abstand d beabstandet ist, wobei der Abstand d kleiner als 2,5 mm, vorteilhaft kleiner als 1 mm und vorzugsweise etwa 0,4 mm ist.

2. Vorrichtung nach Anspruch 1, wobei um das Befestigungselement (5) ein Ring (900) befestigt ist, insbesondere eingerastet ist, wobei der Ring (900) einen axialen Vorsprung (901) aufweist, der mit dem Blockierelement (500) zusammenwirkt, derart, dass der axiale Vorsprung (901) des Rings (900) in der Blockierposition des Blockierelements (500) mit einer axialen Blockierverlängerung (501) des Blockierelements (500) zusammenwirkt, um dadurch eine axiale Verlagerung des Vorratsbehälters (100) zu verhindern, und der axiale Vorsprung (901) des Rings (900) in der Betätigungsposition des Blockierelements (500) mit einer axialen Aussparung (502) des Blockierelements (500) zusammenwirkt, um dadurch eine axiale Verlagerung des Vorratsbehälters (100) zuzulassen.

3. Vorrichtung nach Anspruch 2, wobei der axiale Vorsprung (901) des Rings (900) in der Blockierposition des Blockierelements (500) das Blockierelement (500) in seine Betätigungsposition drängt.

4. Vorrichtung nach Anspruch 2 oder 3, wobei um den Ring (900) ein Schrumpfring (950) aufgeschrumpft ist.

5. Vorrichtung nach einem der vorhergehenden Ansprüche, wobei das Blockierelement (500) einen Verriegelungsvorsprung (510) aufweist, der in der Verriegelungsposition des Auslöserelements (600) mit einer Verriegelungsschulter (610) des Auslöserelements (600) zusammenwirkt, um eine Verschlusseinrichtung zu definieren, die die Verlagerung und/oder die Verformung des Blockierelements aus seiner Blockierposition verhindert.

6. Vorrichtung nach Anspruch 5, wobei die Kraft (F'), die in der Blockierposition durch den Verriegelungsvorsprung (510) des Blockierelements (500) auf die Verriegelungsschulter (610) des Auslöserelements (600) ausgeübt wird, längs einer Achse Z senkrecht zu der Drehachse C des Auslöserelements (600) orientiert ist.

7. Vorrichtung nach Anspruch 6, wobei die Achse Z von der Drehachse C um einen von null verschiedenen Abstand d' beabstandet ist, wobei der Abstand d' kleiner als 2 mm, vorteilhaft kleiner als 1 mm und vorzugsweise etwa 0,25 mm ist.

8. Vorrichtung nach einem der vorhergehenden Ansprüche, wobei die Verschlusseinrichtung in der Verriegelungsposition des Auslöserelements (600) einen ersten Kontaktpunkt zwischen dem Blockierelement (500) und dem Auslöserelement (600) bildet, wobei das Blockierelement (500) einen Abstützvorsprung (520) aufweist, der in der Verriegelungsposition des Auslöserelements (600) mit einer Abstützoberfläche (620) des Auslöserelements (600) zusammenwirkt, um in der Verriegelungsposition des Auslöserelements (600) einen zweiten Kontaktpunkt zwischen dem Blockierelement (500) und dem Auslöserelement (600) zu bilden, wobei der zweite Kontaktpunkt in der Verriegelungsposition des Auslöserelements (600) in einem Abstand von der Achse C des Auslöserelements (600) ist, der größer als der Abstand zwischen der Achse C und dem ersten Kontaktpunkt ist.

9. Vorrichtung nach einem der vorhergehenden Ansprüche, wobei ein seitlicher Betätigungsgriff (20) an dem Körper (10) zwischen einer Ruheposition und einer Verwendungsposition rotatorisch und translatorisch verlagerbar montiert ist, wobei eine Verlagerung des seitlichen Betätigungsgriffs (20) in seine Verwendungsposition das Betätigungsorgan (800) axial in seine gespannte Position verlagert.

10. Vorrichtung nach Anspruch 9, wobei der seitliche Betätigungsgriff (20) eine erste Abstützzone (P1) an dem Betätigungsorgan (800) und eine zweite Abstützzone (P2) an dem Körper (10) aufweist.

11. Vorrichtung nach Anspruch 10, wobei die erste Abstützzone (P1) ein Drehpunkt ist und die zweite Abstützzone (P2) eine radiale Gleitoberfläche ist.

12. Vorrichtung nach einem der Ansprüche 9 bis 11, die ein Blockierorgan (980) aufweist, das zwischen einer blockierenden Position und einer nicht blockierenden Position verlagerbar und/oder verformbar ist, wobei das Blockierorgan (980) in der blockierenden Position mit dem Auslöserelement (600) zusammenwirkt, um dieses daran zu hindern, sich in seine Freigabeposition zu verlagern, wobei der seitliche Betätigungsgriff (20) einen Vorsprung (29) aufweist, der mit dem Blockierorgan (980) zusammenwirkt, wenn der seitliche Betätigungsgriff (20) in seine Verwendungsposition verlagert ist, um das Blockierorgan (980) in seine nicht blockierende Position zu verlagern und/oder zu verformen.

13. Vorrichtung nach einem der vorhergehenden Ansprüche, wobei das auf die Einatmung ansprechende Organ (60, 61) eine verformbare Membran (61) aufweist, die eine verformbare Luftkammer (60) definiert, wobei die verformbare Membran (61) an dem Auslöserelement (600) befestigt ist und wobei die verformbare Membran (61) bei der Einatmung verformt wird, derart, dass sie das Auslöserelement (600) aus seiner Verriegelungsposition in seine Freigabeposition verlagert.

14. Vorrichtung nach einem der vorhergehenden Ansprüche, wobei das Mundstück (400) eine Öffnung (410) aufweist, die mit dem Innenraum des Körpers (10) verbunden ist, wobei die Öffnung (410) zu Beginn der Einatmung durch eine Klappe (420) geschlossen ist, derart, dass der Einatmungsstrom zunächst hauptsächlich an das System für die Auslösung durch Einatmung übertragen wird.

15. Vorrichtung nach Anspruch 14, wobei die Klappe (420) geöffnet wird, wenn sich der Ring (900) axial zusammen mit dem Vorratsbehälter (100) verlagert.

## Claims

1. An inhalation-synchronized fluid dispenser device comprising a body (10) provided with a mouthpiece (400), a fluid reservoir (100) containing a fluid and a propellant gas being mounted to slide axially relative to said body (10), a metering valve (200) including a valve member (210) being assembled by means of a fastener element (5), such as a crimping cap, on said reservoir (100) for selectively dispensing the fluid, said device further comprising:
• a blocking element (500) that pivots about a pivot axis B between a blocking position in which said metering valve (200) cannot be actuated, and an actuation position in which said metering valve (200) can be actuated,
• a trigger element (600) that pivots about a pivot axis C between a locking position in which it blocks said blocking element (500) in its blocking position, and a release position in which it does not block said blocking element (500), and
• an inhalation-controlled trigger system including an inhalation-sensitive member (60, 61) that is deformable and/or movable under the effect of inhaling, said inhalation-sensitive member (60, 61) co-operating with said trigger element (600), so that when said inhalation-sensitive member (60, 61) is deformed and/or moved, it moves and/or deforms said trigger element (600) towards its release position, thereby making it possible to move and/or deform said blocking element (500) from its blocking position towards its actuation position,
said device comprising an actuator member (800) that is mounted to move axially, in particular in sliding, in said body (10) between a rest position and a primed position, and a spring (850) **characterized in that** said spring is arranged between said actuator member (800) and said reservoir (100) or an element (900; 950) that is secured to said reservoir (100), so that when said actuator member (800) moves towards its primed position, said spring (850) is compressed, so as to transmit an axial force (F) to said reservoir (100), said axial force (F) extending along an axis Y that is perpendicular to said pivot axis B of said blocking element (500), said axis Y being spaced apart from said pivot axis B by a distance d that is not zero, said distance d being less than 2.5 mm, advantageously less than 1 mm, preferably about 0.4 mm.

2. A device according to claim 1, wherein a ring (900) is fastened, in particular snap-fastened, around said fastener element (5), said ring (900) including an axial projection (901) that co-operates with said blocking element (500), such that in the blocking position of said blocking element (500), said axial projection (901) of said ring (900) co-operates with an axial blocking extension (501) of said blocking element (500), thereby preventing said reservoir (100) from moving axially, and in the actuation position of said blocking element (500), said axial projection (901) of said ring (900) co-operates with an axial recess (502) of said blocking element (500), thereby enabling said reservoir (100) to move axially.

3. A device according to claim 2, wherein, in the blocking position of said blocking element (500), said axial projection (901) of said ring (900) urges said blocking element (500) towards its actuation position.

4. A device according to claim 2 or claim 3, wherein said hoop (950) is engaged around said ring (900).

5. A device according to any preceding claim, wherein said blocking element (500) includes a locking projection (510) that, in the locking position of the trigger element (600), co-operates with a locking shoulder (610) of said trigger element (600) so as to define a latch that prevents said blocking element (500) from moving and/or deforming out of its blocking position.

6. A device according to claim 5, wherein in the blocking position, the force (F') exerted by said locking projection (510) of said blocking element (500) on said locking shoulder (610) of said trigger element (600) extends along an axis Z that is perpendicular to said pivot axis C of said trigger element (600).

7. A device according to claim 6, wherein said axis Z is spaced apart from said pivot axis C by a distance d' that is not zero, said distance d' being less than 2 mm, advantageously less than 1 mm, preferably about 0.25 mm.

8. A device according to any preceding claim, wherein in the locking position of the trigger element (600), said latch forms a first contact point between said blocking element (500) and said trigger element (600), said blocking element (500) including a bearing projection (520) that, in the locking position of the trigger element (600), co-operates with a bearing surface (620) of said trigger element (600) so as to form, in the locking position of the trigger element (600), a second contact point between said blocking element (500) and said trigger element (600), said second contact point being, in the locking position of the trigger element (600), at a distance from said axis C of the trigger element (600) that is greater than the distance between said axis C and said first contact point.

9. A device according to any preceding claim, wherein a laterally-actuated pusher (20) is mounted to move in pivoting and in translation on said body (10) between a rest position and a working position, movement of said laterally-actuated pusher (20) towards its working position moving said actuator member (800) axially towards its primed position.

10. A device according to claim 9, wherein said laterally-actuated pusher (20) includes a first bearing zone (P1) for bearing against said actuator member (800), and a second bearing zone (P2) for bearing against the body (10).

11. A device according to claim 10, wherein said first bearing zone (P1) is a pivot point, and said second bearing zone (P2) is a surface for radial sliding.

12. A device according to any one of claims 9 to 11, including a blocking member (980) that is movable and/or deformable between a blocking position and a non-blocking position, said blocking member (980), in its blocking position, co-operating with said trigger element (600) so as to prevent it from moving towards its release position, said laterally-actuated pusher (20) including a projection (29) that co-operates with said blocking member (980) when said laterally-actuated pusher (20) is moved towards its working position, so as to move and/or deform said blocking member (980) towards its non-blocking position.

13. A device according to any preceding claim, wherein said inhalation-sensitive member (60, 61) includes a deformable membrane (61) that defines a deformable air chamber (60), said deformable membrane (61) being fastened to said trigger element (600), said deformable membrane (61) being deformed during inhaling, so that it moves said trigger element (600) from its locking position towards its release position.

14. A device according to any preceding claim, wherein said mouthpiece (400) includes an opening (410) that is connected to the inside of the body (10), said opening (410) being closed at the start of inhaling by a check valve (420), such that the inhalation flow due to inhaling initially passes mainly to the trigger system.

15. A device according to claim 14, wherein said check valve (420) is opened when said ring (900) moves axially together with said reservoir (100).
